# EUROPÄISCHE PATENTSCHRIFT

(11) **EP 2 129 297 B1**
(45) Veröffentlichungstag und Bekanntmachung des Hinweises auf die Patenterteilung: **06.10.2010**
(21) Anmeldenummer: 08734608.6
(22) Anmeldetag: 13.03.2008
(51) Int. Cl.: A61B 10/02, A61B 18/08, A61B 17/00, A61B 19/00

(54) **INSTRUMENT FÜR DIE MEDIZINISCHE UNTERSUCHUNG VON ENGEN KÖRPERKANÄLEN**
INSTRUMENT FOR THE MEDICAL EXAMINATION OF TIGHT BODY DUCTS
INSTRUMENT POUR L'EXAMEN MÉDICAL DE CANAUX CORPORELS ÉTROITS

(30) Priorität: 23.03.2007 DE 102007014634
(43) Veröffentlichungstag der Anmeldung: 09.12.2009
(73) Patentinhaber: Riek, Siegfried, 78628 Rottweil (DE); Bachmann, Karl-Heinz, 78667 Villingendorf (DE); Gaiselmann, Thomas, 78667 Villingendorf (DE); Walwiener, Diethelm, 72076 Tübingen (DE)
(72) Erfinder: Riek, Siegfried, 78628 Rottweil (DE); Bachmann, Karl-Heinz, 78667 Villingendorf (DE); Gaiselmann, Thomas, 78667 Villingendorf (DE); Walwiener, Diethelm, 72076 Tübingen (DE)
(74) Vertreter: Modrow, Stephanie
(86) Internationale Anmeldenummer: PCT/EP2008/002002
(87) Internationale Veröffentlichungsnummer: WO 2008/116563

(56) Entgegenhaltungen:
- EP-A- 1 518 498
- DE-A1- 19 906 592
- DE-C1- 19 547 246
- US-A- 5 213 110
- US-A1- 2004 034 278
- US-A1- 2005 070 818
- US-A1- 2006 224 082

## Beschreibung

Die Erfindung betrifft ein Instrument für die medizinische Untersuchung von engen Körperkanälen gemäß dem Oberbegriff der Patentansprüche 1 und 2.

Bei der Duktoskopie werden zur Untersuchung enger Körperkanäle Optiken in den Kanal eingeführt. Zur weiteren Diagnostik können dabei auch Gewebeproben durch Biopsie aus den Körperkanälen entnommen werden. Ein wichtiges Anwendungsgebiet ist dabei die Probeexzision von Gewebe in den Milchgängen (Ductus lactiferi) der weiblichen Brust zur Diagnostik von Milchgangsveränderungen, wie Adenomen, Karzinomvorstufen und Mammakarzinomen.

Wegen des geringen Durchmessers der Milchgänge von maximal etwa 1,2 mm war es lange Zeit nur möglich, ein Instrument für die Probeexzision blind in den Duktus einzuführen und das Instrument in Bezug auf den für die Biopsie vorgesehenen Gewebebereich von außen röntgenologisch oder mittels Ultraschall zu positionieren. Durch die Miniaturisierung der medizinischen Endoskope hat sich inzwischen die Möglichkeit ergeben, eine Probeexzision auch in engen Körperkanälen, wie dem Milchgang der Brust unter Sicht durchzuführen.

Aus der DE 102 58 483 B3 ist hierzu ein Instrument bekannt, welches eine Kanüle mit einem so geringen Außendurchmesser aufweist, dass diese Kanüle in den Milchgang eingeführt werden kann. In der Kanüle ist eine endoskopische Optik mit einem Durchmesser von beispielsweise 1 mm angeordnet. Mittels dieser Optik kann durch die offene oder durch ein durchsichtiges Fenster verschlossene distale Spitze der Kanüle der Bereich des Duktus vor der distalen Spitze der Kanüle beobachtet werden. Die Kanüle weist seitlich hinter der Spitze einen Wanddurchbruch auf, in welchen eine Gewebeprobe eingesaugt oder durch die Gewebespannung eingedrückt wird. Der Rand des Wanddurchbruchs ist als Schneide ausgebildet und durch Verschieben der endoskopischen Optik kann die durch den Wanddurchbruch in das Kanüleninnere eingedrungene Gewebeprobe gegen diese Schneide gedrückt und abgetrennt werden. Bei diesem Instrument ist die Sicht durch die endoskopische Optik dadurch erschwert, dass sich Körpergewebe beim Vorschieben der Kanüle und der Optik an die flache distale Stirnfläche der Optik oder des die Kanüle distal verschließenden Fensters unter axialem Druck anlegt. Durch diesen Druck wird das Gewebe anämisch und erscheint amorph, so dass eine differenzierte Betrachtung des Gewebes erschwert wird. Auch sich vor der Optik bzw. dem distalen Kanülenfenster ansammelnde Körperflüssigkeit oder Blut behindern die Sicht. Schließlich ist bei diesem Instrument das Sichtfeld der endoskopischen Optik auf den distal vor der Spitze liegenden Bereich beschränkt. Die Vorwärtsbewegung der Spitze des Instruments durch den Kanal ist schwer zu beurteilen und somit eine Orientierung im Kanal beeinträchtigt. Die Positionierung des Wanddurchbruchs auf den zu entnehmenden Gewebebereich kann dann nur noch geschätzt werden und nicht unter unmittelbarer Sicht erfolgen. Der eigentliche Vorgang der Gewebeentnahme kann nicht unter Sicht erfolgen, da hierbei die Optik gegen das Gewebe gepresst und durch Blut bedeckt wird.

Aus der DE 100 35 878 A1 ist ein Instrument für die Biopsie bekannt, welches aus einem Miniaturgreifinstrument mit koaxialer Optik besteht. Auch bei diesem Instrument treten die genannten Nachteile beim Einführen einer planen distalen Stirnfläche der Optik in den Kanal auf. Nach der Gewebeentnahme wird die Sicht der Optik vollständig durch das Biopsat und Blut verdeckt.

Aus EP 1518 498 A1 ist ein Instrument nach dem Oberbegriff der Patentansprüche 1 und 2 bekannt.

Aus US 2004/34 278 A1 und DE 195 47 246 C1 sind auch andere Instrumente für die medizinische Untersuchung von engen Körperkanälen bekannt.

Der Erfindung liegt die Aufgabe zu Grunde, ein Instrument für die Untersuchung enger Körperkanäle so zu verbessern, dass eine Inspektion und gegebenenfalls eine Probeexzision unter guter Sicht möglich sind.

Diese Aufgabe wird erfindungsgemäß gelöst durch ein Instrument mit den Merkmalen der Patentansprüche 1 oder 2.

Vorteilhafte Ausführungen und Weiterbildungen der Erfindung sind in den Unteransprüchen angegeben.

Bei dem erfindungsgemäßen Instrument für die Untersuchung enger Körperkanäle, insbesondere der Milchggänge der Brust ist am distalen Ende einer Kanüle eine distal ausgewölbte Spitze angeordnet, die durchsichtig ausgebildet ist. An diese gewölbte distale Spitze schließt sich in der zylindrischen Wandung der Kanüle wenigstens eine seitliche Vertiefung der äußeren Umfangsfläche an. Die Vertiefung ist gegen das kanüleninnere geschlossen. Auch im Bereich dieser Vertiefung ist das distale Ende der Kanüle durchsichtig ausgebildet. In die Kanüle wird eine endoskopische Optik eingesetzt, wobei die axiale Position des distalen Endes der Optik und deren optische Eigenschaften so gewählt werden, dass das Sichtfeld dieser Optik sowohl die gewölbte Spitze als auch den anschließenden zylindrischen Bereich mit der Vertiefung erfasst.

Beim Einführen des Instruments in einen Duktus des Körpers, z.B. in einen Milchgang, weitet die vorgewölbte Spitze den Duktus auf, so dass das Körpergewebe seitlich an der sich vorwärts bewegenden Spitze entlang gleitet, ohne dass sich ein axialer Druck des Gewebes vor der Spitze aufbauen kann. Auch in dem Duktus enthaltene Körperflüssigkeit strömt an der gewölbten Spitze seitlich vorbei. Mittels der endoskopischen Optik kann das an der Spitze und dem anschließenden durchsichtigen zylindrischen Bereich der Kanüle vorbeigleitende Gewebe nahezu druckfrei beobachtet werden, so dass Gewebestrukturen gut sichtbar und differenzierbar sind. Der Vorschub des Instruments und seiner distalen Spitze in dem Kanal kann dadurch gut beurteilt werden. Das distale Ende der Kanüle kann auf diese Weise unter differenzierter Beobachtung des an der Spitze und dem anschließenden durchsichtigen Bereich anliegenden Gewebes so positioniert werden, dass ein auffälliger Gewebebereich an der seitlichen Vertiefung anliegt und in diese eintritt. Beim Eintritt in die Vertiefung kann sich das Gewebe praktisch druckfrei entspannen, so dass es ohne ischämische Verfärbung oder Quetschung inspiziert und beurteilt werden kann. Da die Vertiefung gegen das Kanüleninnere geschlossen ist, gelangt auch keine Körperflüssigkeit oder Blut in das Kanülenlumen vor die Optik, wodurch die Sicht behindert werden könnte.

In einer bevorzugten Ausführung des Instruments kann zusätzlich auf dem Außenumfang der Kanüle ein Messer angeordnet sein, das auf der Kanüle geführt über die Vertiefung hinweg bewegt werden kann. Für eine Biopsie kann das Messer betätigt werden, um das sich in der Vertiefung befindende Gewebe abzutrennen. Das abgetrennte Gewebe wird dabei durch das über die Vertiefung geführte Messer in dieser Vertiefung gehalten, so dass es durch Herausziehen des Instruments für eine anschließende Untersuchung entnommen werden kann. Das Abtrennen des Gewebes kann dabei durch die Optik beobachtet werden. Auch das in der Vertiefung aufgenommene abgetrennte Biopsat kann beobachtet werden, ohne dass Blut die Optik beeinträchtigt.

Eine besonders gute Beobachtung des die distale Spitze umgebenden Gewebes ergibt sich, wenn die distale Spitze als hohle gewölbte Kuppel ausgebildet ist, deren Mittelachse mit der Mittelachse der Kanüle zusammenfällt.

Das Messer ist auf der Kanüle axial oder rotatorisch bewegbar und in einer vorteilhaften Ausführung als Ringmesser ausgebildet, welches die Kanüle koaxial umschließt und axial auf der Kanüle bewegbar ist. Diese Ausführung ermöglicht eine besonders einfache Betätigung des Messers durch einen am proximalen Ende des Instruments angeordneten Betätigungsgriff.

Das Ringmesser ist dabei vorzugsweise mit einer distalen Schneide ausgebildet und kann zwischen einer proximalen und einer distalen Endstellung bewegt werden, wobei in der proximalen Endstellung die Schneide des Ringmessers sich proximal hinter der Vertiefung befindet und in distaler Richtung über die Vertiefung hinweg in die distale Endstellung vor der Vertiefung bewegt wird. Dabei kann eine radiale Ringschulter als Anschlag für das Ringmesser dienen.

Da die Vertiefung in der Wandung des distalen Endes der Kanüle ausgebildet ist, weist die Vertiefung nur eine relativ geringe radiale Tiefe auf. Um eine ausreichende Menge des Gewebematerials zu gewinnen, ist die Vertiefung zweckmäßig als axial verlaufende Rinne ausgebildet, um die Aufnahmekapazität der Vertiefung zu vergrößern.

Die Vertiefung kann in unterschiedlicher Weise hergestellt werden. Wesentlich ist, dass die Vertiefung in der äußeren Mantelfläche des Kanülenendes ausgebildet ist, d. h. nach außen offen und gegen das innere Kanülenlumen geschlossen ist.

Die Wandung kann durch Verformung radial in das Kanüleninnere gedrückt werden, um die Vertiefung zu bilden. Alternativ wäre es auch möglich, in die Wandung einen Durchbruch einzubringen und in diesen Durchbruch einen Topf einzusetzen und zu verkleben, zu verschweißen usw., der dann die Vertiefung bildet.

In einer Weiterbildung der Erfindung ist auf der Außenfläche des Messers oder des Kanülenendes eine Koagulationseinrichtung angebracht, die vorzugsweise als elektrisch beheizbare Koagulationszone ausgebildet ist. Ist mittels des Messers eine Gewebeprobe abgetrennt, so kann die Koagulationseinrichtung in den Bereich der Schnittwunde gebracht werden, um die beim Abtrennen der Gewebeprobe verletzten Gefäße zu koagulieren. Die Koagualtionseinrichtung kann so auf dem Messer angeordnet sein, dass sie beim Abtrennen des Gewebes in den Bereich der Schnittwunde gelangt, also mit der dann durch das Messer verschlossenen Vertiefung zur Deckung kommt. Dies macht eine zusätzliche Positionierung der Koagulationseinrichtung unnötig. In einer anderen Ausführung ist die Koagulationseinrichtung in axialer oder rotatorischer Richtung um einen definierten und damit gezielt nachführbaren Weg versetzt gegenüber der Vertiefung angeordnet. Dadurch kann ausgeschlossen werden, dass die Hitze der Koagulationseinrichtung die in der Vertiefung aufgenommene und eingeschlossene Gewebeprobe schädigt.

Der Kanülenschaft kann je nach Anwendungszweck des Instruments als starrer Schaft, z.B. in Form eines Metallrohres ausgebildet sein oder als elastisch flexibler Schaft, z.B. in Form eines Kunststoffrohres. Bei der Ausbildung des Kanülenschaftes als Metallrohr ist das durchsichtige distale Ende, welches die Spitze und den Zylinderbereich mit der Vertiefung umfasst, vorzugsweise aus einem durchsichtigen Kunststoff, insbesondere einstückig gefertigt und an dem Kanülenschaft befestigt. Ist der Kanülenschaft als Kunststoffrohr ausgebildet, so kann das distale Ende mit der Spitze und der Vertiefung in gleicher Weise ausgebildet und an diesem Kunststoffrohr befestigt werden. Ebenso ist es möglich, das Kunststoffrohr des Kanülenschaftes und das distale Ende einstückig aus durchsichtigem Kunststoff zu fertigen.

Im Übrigen kann in dem Kanülenschaft zusätzlich ein Spülkanal angeordnet sein. Erforderlichenfalls kann in dem Kanülenschaft auch noch ein Saugkanal angeordnet oder ausgebildet sein, der in die Vertiefung des distalen Endes der Kanüle mündet. Mittels eines Unterdrucks in diesem Saugkanal kann das zu entnehmende Gewebe zusätzlich in die Vertiefung eingesaugt werden. Diese Spül- bzw. Saugkanäle können auch in dem Raum zwischen dem Kanülenschaft und dem Messer ausgebildet sein.

In einer Weiterbildung der Erfindung ist es möglich, auf das distale Ende der Kanüle einen Markierungsring aufzuschieben. Dieser Markierungsring kann in dem Duktus an der Stelle, an welcher die Probeexzision durchgeführt wird, von dem distalen Ende der Kanüle abgestreift werden, so dass er an dieser Stelle in dem Duktus verbleibt, um diese Stelle zu markieren. Der Markierungsring besteht dabei aus einem Material, welches den Markierungsring röntgenologisch oder mittels Ultraschall erkennbar macht. Durch einen solchen an der Stelle der Probeexzision deponierten Markierungsring kann ein bei der histologischen Untersuchung des entnommenen Gewebes eventuell diagnostizierter Tumor bei einem nachfolgenden operativen Eingriff leicht gefunden werden. Zum Abstreifen des Markierungsrings kann gegebenenfalls die distale Vorschubbewegung des Ringmessers ausgenützt werden.

Im Folgenden wird die Erfindung anhand eines in der Zeichnung dargestellten Ausführungsbeispiels näher erläutert. Es zeigen
- Fig. 1: eine Seitenansicht des distalen Endes des Instrumen- tes,
- Fig. 2: eine um 90° gegenüber Figur 1 gedrehte Ansicht und
- Fig. 3: einen Querschnitt gemäß der Schnittlinie A-A in Figur 1.

Das in der Zeichnung dargestellte Ausführungsbeispiel zeigt ein Instrument für die Duktoskopie und insbesondere für die Probeexzision im Milchgang der weiblichen Brust.

In der Zeichnung ist nur das distale Ende des Instruments dargestellt. Die nicht dargestellten Teile des Instruments entsprechen herkömmlichem Stand der Technik, so dass diese nicht zeichnerisch dargestellt und näher beschrieben werden müssen.

Das Instrument weist eine Kanüle mit einem Kanülenschaft 6 auf, an dessen nicht dargestelltem proximalen Ende ein Griff angebracht ist. Der Kanülenschaft 6 kann als starres Rohr, insbesondere als Metallrohr oder als elastisch flexibles Rohr, insbesondere als Kunststoffrohr ausgebildet sein. Das sich an den Kanülenschaft 6 anschließende distale Ende 1 der Kanüle ist aus einem durchsichtigen Kunststoff, vorzugsweise einstückig hergestellt.

Das distale Ende 1 besteht aus einer distalen Spitze 1a und einem sich daran anschließenden zylindrischen Rohrabschnitt 1b, der an den Kanülenschaft 6 anschließt und mit welchem das Kanülenende 1 in den Kanülenschaft 6 übergeht. Die distale Spitze 1a ist als hohle in distaler Richtung ausgewölbte Kuppel ausgebildet und insbesondere hat die Spitze 1a die Form einer konischen, bauchig ausgewölbten Kuppel, die zu ihrer Mittelachse rotationssymmetrisch ist, wobei die Mittelachse der Spitze 1a mit der Mittelachse des Kanülenschaftes 6 zusammenfällt. Die Spitze 1a geht in den Rohrabschnitt 1b über. Der Rohrabschnitt 1b ist als hohler Kreiszylinder ausgebildet, dessen Außendurchmesser mit dem Außendurchmesser des Kanülenschafts 6 übereinstimmt. Der Außendurchmesser der distalen Spitze 1a ist am Übergang zu dem Rohrabschnitt 1b etwas größer als der Außendurchmesser dieses Rohrabschnittes 1b, so dass hierdurch am Außenumfang des Kanülenendes 1 eine radiale Ringschulter 1c gebildet wird.

In der äußeren Mantelfläche des Rohrabschnittes 1b ist eine Vertiefung 4 ausgebildet, die die Form einer axial verlaufenden Rinne aufweist. Die Vertiefung 4 erstreckt sich im Wesentlichen über die gesamte axiale Länge des Rohrabschnittes 1b. Die radiale Tiefe der Vertiefung 4 entspricht nahezu der Wandstärke des Rohrabschnittes 1b.

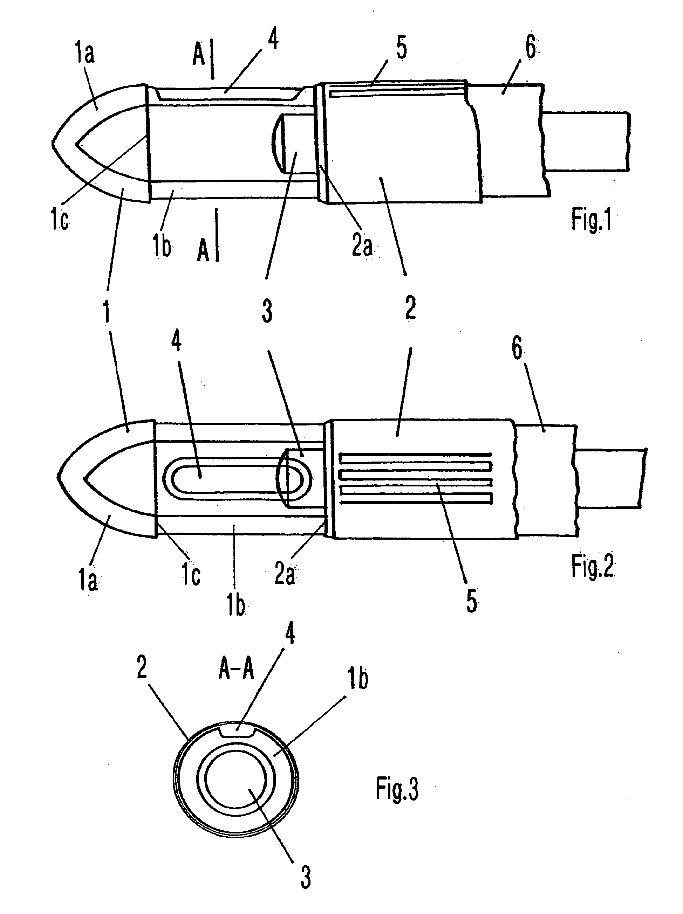

Auf dem Kanülenschaft 6 ist ein Messer 2 angeordnet. Das Messer 2 ist in dem dargestellten Ausführungsbeispiel ein Ringmesser, welches in Form einer Hülse den Kanülenschaft 6 koaxial umschließt. Das Messer 2 ist auf dem Kanülenschaft 6 axial verschiebbar, wozu das Messer 2 mittels eines in der Zeichnung nicht dargestellten am proximalen Ende des Instruments angeordneten Betätigungsgriffes bewegt wird. Die distale Randkante des Messers 2 ist als Schneide 2a ausgebildet. Das Messer 2 ist mittels des Betätigungsgriffes zwischen einer in den Figuren 1 und 2 gezeigten proximalen Endstellung und einer distalen Endstellung verschiebbar. In der proximalen Endstellung befindet sich die Schneide 2a proximal hinter der Vertiefung 4. In der distalen Endstellung ist das Messer 2 soweit nach vorne geschoben, dass die Schneide 2a distal vor der Vertiefung 4 liegt und an der Ringschulter 1c anschlägt. In der distalen Endstellung überdeckt die Hülse des Messers 2 die Vertiefung vollständig.

Auf der Außenfläche der Hülse des Messers 2 ist eine Koagulationseinrichtung angeordnet, die als elektrisch beheizbare Zone 5 ausgebildet ist. Die beheizbare Zone 5 kann beispielsweise durch einen auf der Hülse des Messers 2 aufgebrachten Heizdraht realisiert sein. Die Fläche der beheizbaren Zone 5 ist so angeordnet und dimensioniert, dass sie bei in die distale Endstellung vorgeschobenem Messer 2 mit der Vertiefung 4 zusammenfällt und diese vollständig überdeckt.

In den Kanülenschaft 6 ist eine endoskopische Optik 3 eingeführt. Die Optik 3 endet distal im Bereich des proximalen Endes des Rohrabschnittes 1b, d.h. am proximalen Ende der Vertiefung 4. Die Optik 3 ist als Weitwinkeloptik ausgebildet, so dass mittels der Optik 3 die gesamte Innenfläche des distalen Kanülenendes 1 also sowohl die hohle ausgewölbte distale Spitze 1a als auch der Rohrabschnitt 1b mit der Vertiefung 4 beobachtet werden können.

Die Funktionsweise des Instruments ist Folgende:
Das Instrument wird in einen engen Duktus des Körpers z.B. in einen Milchgang eingeführt. Dabei befindet sich das Messer 2 in der proximalen Endstellung, so dass das gesamte distale Kanülenende 1 einschließlich der Vertiefung 4 von dem Messer 2 freigegeben sind. Beim Einführen des Instruments wird das durchsichtige distale Kanülenende 1 in dem Duktus vorgeschoben, wobei das Gewebe der Wände des Duktus an der sich erweiternden distalen Spitze 1a und dem anschließenden zylindrischen Rohrabschnitt 1b entlang gleitet und mittels der Optik 3 differenziert beobachtet werden kann. Wird ein interessierender Gewebebereich, z.B. ein Tumor-verdächtiger Gewebebereich durch das durchsichtige Kanülenende 1 erkannt, so wird das Kanülenende 1 unter Sicht durch die Optik 3 so positioniert, dass sich die Vertiefung 4 an diesem Gewebebereich befindet. Das Gewebe wird dabei durch seine Eigenspannung in die Vertiefung 4 gedrückt. Gegebenenfalls kann dies durch einen Unterdruck unterstützt werden. Nun kann das in der Vertiefung liegende Gewebe praktisch druckfrei mittels der Optik 3 inspiziert und beurteilt werden. Wird dabei erkannt wird, dass das sich in der Vertiefung 4 befindende Gewebe näher untersucht werden muss, so wird das Messer 2 betätigt und schiebt sich über den Rohrabschnitt 1b und die Vertiefung 4 distal nach vorne. Die Schneide 2a des Messers 2 trennt dabei das sich in der Vertiefung 4 befindende Gewebe ab, was gegebenenfalls noch durch den Anschlag der Schneide 2a an der Ringschulter 1c begünstigt wird. Das abgetrennte Gewebe ist nun durch die Ringhülse des Messers 2 in der Vertiefung 4 eingeschlossen. Die beheizbare Zone 5 kann nun an den Gewebebereich gebracht werden, an welchem die Gewebeprobe abgetrennt wurde. Durch Betätigung der elektrischen Heizung kann die durch das Abtrennen der Gewebeprobe verursachte Wunde koaguliert werden, um insbesondere ein Nachbluten zu verhindern. Anschließend wird das Instrument herausgezogen und die in der Vertiefung 4 eingeschlossene Gewebeprobe kann für die histologische Untersuchung entnommen werden.

### Bezugszeichenliste

- 1: distales Kanülenende
- 1a: distale Spitze
- 1b: Rohrabschnitt
- 1c: Ringschulter
- 2: Messer
- 2a: Schneide
- 3: endoskopische Optik
- 4: Vertiefung
- 5: Koagulationszone
- 6: Kanülenschaft

## Patentansprüche

1. Instrument für die medizinische Untersuchung von engen Körperkanälen, insbesondere der Milchgänge der Brust, mit einer Kanüle, die einen Kanülenschaft (6) und eine durchsichtige konische distale Spitze (1a) aufweist, mit wenigstens einer seitlich in der Kanüle hinter der distalen Spitze angeordneten Aufnahme für eine Gewebeprobe und mit einer in das Kanüleninnere einführbaren endoskopischen Optik (3), wobei die seitliche Aufnahme als wenigstens eine Vertiefung (4) in der äußeren Mantelfläche der Kanüle ausgebildet ist und wobei das distale Kanülenende (1) von der Spitze (1a) bis zumindest einschließlich der seitlichen Vertiefung (4) durchsichtig ist, sodass das Sichtfeld der eingeführten Optik (3) das distale Kanülenende (1) einschließlich der Vertiefung (4) erfasst,
**dadurch gekennzeichnet, dass**
die distale Spitze (1a) des Kanülenendes (1) als hohle in distaler Richtung ausgewölbte Kuppel ausgebildet ist, deren Mittelachse mit der Achse des Kanülenschaftes (6) zusammenfällt und dass die wenigstens eine Vertiefung (4) gegen das Kanüleninnere geschlossen ist und als radiale Verformung der Wandung des Kanülenendes (1) ausgebildet ist.

2. Instrument für die medizinische Untersuchung von engen Körperkanälen, insbesondere der Milchgänge der Brust, mit einer Kanüle, die einen Kanülenschaft (6) und eine durchsichtige konische distale Spitze (1a) aufweist, mit wenigstens einer seitlich in der Kanüle hinter der distalen Spitze angeordneten Aufnahme für eine Gewebeprobe und mit einer in das Kanüleninnere einführbaren endoskopischen Optik (3), wobei die seitliche Aufnahme als wenigstens eine Vertiefung (4) in der äußeren Mantelfläche der Kanüle ausgebildet ist und wobei das distale Kanülenende (1) von der Spitze (1a) bis zumindest einschließlich der seitlichen Vertiefung (4) durchsichtig ist, sodass das Sichtfeld der eingeführten Optik (3) das distale Kanülenende (1) einschließlich der Vertiefung (4) erfasst,
**dadurch gekennzeichnet, dass**
die distale Spitze (1a) des Kanülenendes (1) als hohle in distaler Richtung ausgewölbte Kuppel ausgebildet ist, deren Mittelachse mit der Achse des Kanülenschaftes (6) zusammenfällt und dass die wenigstens eine Vertiefung (4) gegen das Kanüleninnere geschlossen ist und durch einen in einen Ausbruch der Wandung des Kanülenendes (1) eingesetzten Topf gebildet ist.

3. Instrument nach Anspruch 1,
**dadurch gekennzeichnet, dass**
die radiale Verformung in das Kanüleninnere gedrückt ist.

4. Instrument nach einem der Ansprüche 1-3,
**dadurch gekennzeichnet, dass**
ein Messer (2) auf der Kanüle geführt über die Vertiefung (4) bewegbar ist, wozu das Messer (2) die Kanüle koaxial umschließt und auf einem zylindrischen Rohrabschnitt (1b) des Kanülenendes (1), in welchem die Vertiefung (4) ausgebildet ist, axial oder in Umfangsrichtung rotatorisch bewegbar ist.

5. Instrument nach Anspruch 4,
**dadurch gekennzeichnet, dass**
das Messer (2) als Ringmesser ausgebildet ist, eine distale Schneide (2a) aufweist und zwischen einer proximalen und einer distalen Endstellung axial bewegbar ist, wobei sich die Schneide (2a) in der proximalen Endstellung proximal von der Vertiefung (4) und in der distalen Endstellung distal von der Vertiefung (4) befindet.

6. Instrument nach einem der vorhergehenden Ansprüche,
**dadurch gekennzeichnet, dass**
das distale Kanülenende (1) von der Spitze (1a) bis ein schließlich des die Vertiefung (4) aufweisenden Rohrabschnittes (1b) aus einem durchsichtigen Kunststoff einstückig gefertigt ist.

7. Instrument nach einem der vorhergehenden Ansprüche,
**dadurch gekennzeichnet, dass**
das Kanülenende (1) oder das Messer (2) auf der Außenfläche wenigstens eine elektrisch beheizbare Koagulationseinrichtung aufweist.

8. Instrument nach Anspruch 7,
**dadurch gekennzeichnet, dass**
die Koagulationseinrichtung durch die Bewegung des Messers (2) und/oder der Kanüle in den Bereich der vorhergehenden Probeexzision (4) bewegbar ist.

9. Instrument nach Anspruch 8,
**dadurch gekennzeichnet, dass**
die Koagulationseinrichtung so auf der Außenfläche des Messers (2) angeordnet ist, dass sie mit der Vertiefung (4) zur Flächendeckung kommt, wenn das Messer (2) über die Vertiefung (4) bewegt wird.

10. Instrument nach einem der vorhergehenden Ansprüche,
**dadurch gekennzeichnet, dass**
in dem Kanülenschaft (6) ein Spülkanal angeordnet ist.

11. Instrument nach einem der vorhergehenden Ansprüche,
**dadurch gekennzeichnet, dass**
in dem Kanülenschaft (6) ein in die Vertiefung (4) mündender Saugkanal angeordnet ist.

12. Instrument nach einem der vorhergehenden Ansprüche,
**dadurch gekennzeichnet, dass**
der Raum zwischen dem Kanülenschaft (6) und dem Messer (2) als Spülkanal und/oder Saugkanal ausgebildet ist.

13. Instrument nach einem der vorhergehenden Ansprüche,
**dadurch gekennzeichnet, dass**
auf dem distalen Kanülenende (1) ein Markierungsring aufbringbar ist, der zum Verbleib in dem Körperkanal von dem Kanülenende (1) abgestreift werden kann.

## Claims

1. Instrument for the medical examination of narrow body ducts, in particular the milk ducts of the breast, comprising a cannula which has a cannula shaft (6) and a transparent conical distal tip (1a), comprising at least one holder for a tissue sample which is arranged laterally in the cannula behind the distal tip, and comprising an endoscopic lens (3) which can be inserted into the interior of the cannula, wherein the lateral holder is designed as at least one depression (4) in the outer jacket surface of the cannula and wherein the distal cannula end (1) from the tip (1a) as far as at least including the lateral depression (4) is transparent so that the field of vision of the inserted lens (3) covers the distal cannula end (1) including the depression (4), **characterised in that** the distal tip (1a) of the cannula end (1) is designed as a hollow dome which is curved in the distal direction, the central axis of which coincides with the axis of the cannula shaft (6), and **in that** the at least one depression (4) is closed towards the interior of the cannula and is designed as a radial deformation of the wall of the cannula end (1).

2. Instrument for the medical examination of narrow body ducts, in particular the milk ducts of the breast, comprising a cannula which has a cannula shaft (6) and a transparent conical distal tip (1a), comprising at least one holder for a tissue sample which is arranged laterally in the cannula behind the distal tip, and comprising an endoscopic lens (3) which can be inserted into the interior of the cannula, wherein the lateral holder is designed as at least one depression (4) in the outer jacket surface of the cannula and wherein the distal cannula end (1) from the tip (1a) as far as at least including the lateral depression (4) is transparent so that the field of vision of the inserted lens (3) covers the distal cannula end (1) including the depression (4), **characterised in that** the distal tip (1a) of the cannula end (1) is designed as a hollow dome which is curved in the distal direction, the central axis of which coincides with the axis of the cannula shaft (6), and **in that** the at least one depression (4) is closed towards the interior of the cannula and is formed by a container set into a breach in the wall of the cannula end (1).

3. Instrument according to claim 1, **characterised in that** the radial deformation is pushed into the interior of the cannula.

4. Instrument according to one of claims 1-3, **characterised in that** a blade (2) can be moved over the depression (4) in a manner guided on the cannula, for which purpose the blade (2) coaxially surrounds the cannula and can be moved axially or in rotation in the circumferential direction on a cylindrical tubular section (1b) of the cannula end (1) in which the depression (4) is formed.

5. Instrument according to claim 4, **characterised in that** the blade (2) is designed as an annular blade, has a distal cutting edge (2a) and is movable axially between a proximal and a distal end position, wherein the cutting edge (2a) is located proximal to the depression (4) in the proximal end position and distal to the depression (4) in the distal end position.

6. Instrument according to one of the preceding claims, **characterised in that** the distal cannula end (1) from the tip (1a) as far as and including the tubular section (1b) which has the depression (4) is made in one piece from a transparent plastic.

7. Instrument according to one of the preceding claims, **characterised in that** the cannula end (1) or the blade (2) has on the outer surface at least one electrically heatable coagulation device.

8. Instrument according to claim 7, **characterised in that** the coagulation device can be moved into the region of the previous sample excision (4) by moving the blade (2) and/or the cannula.

9. Instrument according to claim 8, **characterised in that** the coagulation device is arranged on the outer surface of the blade (2) in such a way that it covers the surface area of the depression (4) when the blade (2) is moved over the depression (4).

10. Instrument according to one of the preceding claims, **characterised in that** a rinsing channel is arranged in the cannula shaft (6).

11. Instrument according to one of the preceding claims, **characterised in that** a suction channel which opens into the depression (4) is arranged in the cannula shaft (6).

12. Instrument according to one of the preceding claims, **characterised in that** the space between the cannula shaft (6) and the blade (2) is designed as a rinsing channel and/or suction channel.

13. Instrument according to one of the preceding claims, **characterised in that** a marking ring can be applied to the distal cannula end (1), which marking ring can be stripped from the cannula end (1) in order to remain in the body duct.

## Revendications

1. Instrument pour l'examen médical de canaux corporels étroits, en particulier des canaux galactophores du sein comportant une canule qui comporte une tige de canule (6) et une pointe distale conique transparente (1a), au moins un logement de réception pour un échantillon de tissu disposé latéralement dans la canule en arrière de sa pointe distale et une optique endoscopique (3) pouvant être introduite à l'intérieur de la canule, le logement de réception latéral étant réalisé sous la forme d'au moins un évidemment (4) dans la surface enveloppe extérieure de la canule, et l'extrémité distale de la canule étant transparente de la pointe (1a) jusqu'à au moins l'évidemment latéral y compris cet évidemment de sorte que le champ de vision de l'optique (3) introduite détecte l'extrémité distale de la canule (1) y compris l'évidemment (4),
**caractérisé en ce que**
la pointe distale (1a) de l'extrémité de la canule (1) est réalisée sous la forme d'un dôme creux vouté dans la direction distale, dont l'axe médian coïncide avec l'axe de la tige de la canule, et **en ce que** l'évidemment (4) est fermé vis-à-vis de l'intérieur de la canule et est réalisé sous la forme d'une déformation radiale de la paroi de l'extrémité de la canule (1).

2. Instrument pour l'examen médical de canaux corporels étroits, en particulier des canaux galactophores du sein, comportant une canule qui comporte une tige de canule (6) et une pointe distale conique transparente (1a), au moins un logement de réception pour un échantillon de tissu disposé latéralement dans la canule en arrière de sa pointe distale et une optique endoscopique (3) pouvant être introduite à l'intérieur de la canule, le logement de réception latéral étant réalisé sous la forme d'au moins un évidemment (4) dans la surface enveloppe extérieure de la canule, et l'extrémité distale de la canule (1) étant transparente de la pointe (1a) jusqu'à au moins l'évidemment latéral (4) y compris cet évidemment de sorte que le champ de vision de l'optique (3) introduite détecte l'extrémité distale de la canule (1), y compris l'évidemment (4),
**caractérisé en ce que**
la pointe distale (1a) de l'extrémité de la canule (1) est réalisée sous la forme d'un dôme creux vouté dans la direction distale, dont l'axe médian coïncide avec l'axe de la tige (6) de la canule, et **en ce que** l'évidemment (4) est fermée vis-à-vis de l'intérieur de la canule et est formé par un creuset mis en place dans une cavité de la paroi de l'extrémité de la canule (1).

3. Instrument selon la revendication 1,
**caractérisé en ce que**
la déformation radiale est repoussée à l'intérieur de la canule.

4. Instrument selon l'une des revendications 1-3,
**caractérisé en ce que**
un couteau (2) est mobile au dessus de l'évidemment (4) en étant guidé sur la canule, le couteau (2) entourant, à cet effet, coaxialement la canule est étant mobile axialement ou en rotation en direction périphérique sur un tronçon tubulaire cylindrique (1b) de l'extrémité de la canule (1) sur lequel est réalisé l'évidemment (4).

5. Instrument selon la revendication 4,
**caractérisé en ce que**
le couteau (2) est réalisé sous la forme d'un couteau annulaire, comporte une lame distale (2a), et est mobile axialement entre une position d'extrémité proximale et une position d'extrémité distale, dans sa position d'extrémité proximale, la lame (2a) étant située dans une position proximale par rapport à l'évidemment (4), tandis qu'elle est située dans une position distale par rapport à l'évidemment (4) dans sa position d'extrémité distale.

6. Instrument selon l'une des revendications précédentes,
**caractérisé en ce que**
l'extrémité distale de la canule (1) est réalisée en une seule pièce, en un matériau synthétique transparent, de la pointe (1a) jusqu'au tronçon tubulaire (1b) présentant l'évidemment (4) y compris cet évidemment.

7. Instrument selon l'une des revendications précédentes,
**caractérisé en ce que**
l'extrémité de la canule (1) ou la lame (2) comporte sur sa surface extérieure au moins un dispositif de coagulation pouvant être chauffé électriquement.

8. Instrument selon la revendication 7,
**caractérisé en ce que**
le dispositif de coagulation peut être déplacé par le mouvement du couteau (2) et/ou de la canule dans la zone d'une biopsie (4) antécédente.

9. Instrument selon la revendication 8,
**caractérisé en ce que**
le dispositif de coagulation est disposé sur la surface extérieure du couteau (2) de sorte qu'il recouvre la surface de l'évidemment (4) lorsque le couteau (2) est déplacé sur cet évidemment.

10. Instrument selon l'une des revendications précédentes,
**caractérisé en ce que**
un canal de rinçage est disposé dans la tige (6) de la canule.

11. Instrument selon l'une des revendications précédentes,
**caractérisé en ce qu'**
un canal d'aspiration débouchant dans l'évidemment (4) est disposé dans la tige (6) de la canule.

12. Instrument selon l'une des revendications précédentes,
**caractérisé en ce que**
l'espace compris entre la tige (6) de la canule et le couteau (2) est réalisé sous la forme d'un canal de rinçage et/ou d'un canal d'aspiration.

13. Instrument selon l'une des revendications précédentes,
**caractérisé en ce que**
sur l'extrémité distale de la canule (1) on peut mettre en place un anneau repère pouvant être enlevé de cette extrémité pour rester dans le canal corporel.
